# EUROPÄISCHE PATENTANMELDUNG

(11) **EP 4 177 214 A1**
(43) Veröffentlichungstag der Anmeldung: **10.05.2023**
(21) Anmeldenummer: 22205730.9
(22) Anmeldetag: 07.11.2022
(51) Int. Cl.: B67C 3/00

(54) **VERFAHREN ZUM ÜBERWACHEN EINER BEHÄLTERBEHANDLUNGSANLAGE**

(30) Priorität: 09.11.2021 DE 102021129056
(71) Anmelder: KRONES AG, 93073 Neutraubling (DE)
(72) Erfinder: HUBER, Anton, 93073 Neutraubling (DE)
(74) Vertreter: v. Bezold & Partner Patentanwälte - PartG mbB

(57) **Zusammenfassung**

Die Erfindung betrifft ein Verfahren zum Überwachen einer Behälterbehandlungsanlage (10), die eine Füllvorrichtung (12) zum Befüllen von Behältern (28) und eine Verschließvorrichtung (14) zum Verschließen von Behältern (28) aufweist. Das Verfahren weist ein Füllen eines Behälters (28) mit einem Füllgut mittels der Füllvorrichtung (12) und ein Messen einer Konzentration eines Gases in dem Füllgut des gefüllten Behälters (28) mittels eines Messgeräts (32) auf, wobei der gefüllte Behälter (28) zum Messen der Konzentration stromaufwärts von der Verschließvorrichtung (14) entnommen wird oder die Konzentration stromaufwärts von der Verschließvorrichtung (14) gemessen wird. Vorteilhaft ermöglicht das Verfahren, dass zielgerichtet die sauerstoffarme Abfüllung durch die Füllvorrichtung (10) überwacht werden kann.

## Beschreibung

### Technisches Gebiet

Die Erfindung betrifft ein Verfahren zum Überwachen einer Behälterbehandlungsanlage, die eine Füllvorrichtung und eine Verschließvorrichtung aufweist.

### Technischer Hintergrund

Verfahren und Füllvorrichtungen zum Abfüllen von sauerstoffempfindlichen Flüssigkeit, wie z. B. Bier, in Flaschen oder Dosen sind an sich bekannt. Beispielsweise können sauerstoffempfindliche Produkte in einem Füllprozess abgefüllt werden, bei dem die Behälter nacheinander evakuiert, gespült, evakuiert, vorgespannt, gefüllt und entlastet werden. Zusätzliche Evakuierungen sind optional ebenfalls möglich.

Die EP 2 629 093 A1 offenbart ein Verfahren sowie eine Vorrichtung zur Bestimmung des CO2-Gehalts in einer zu prüfenden Flüssigkeit, insbesondere in einem Getränk.

Die DE 102 13 076 A1 offenbart ein Verfahren zur Bestimmung der Gehaltsmengen von in einer Flüssigkeit, vorzugsweise in einem Getränk, gelösten Gasen, wobei nach vollständigem Befüllen einer zumindest mit einem Druckmess-Sensor ausgestatteten Messkammer mit der auf ihren Gasgehalt zu prüfenden Flüssigkeit ("Probeflüssigkeit") und nach fluiddichtem Schließen der Messkammer deren Volumen - von einem Standardvolumen ausgehend - um einen vorgegebenen Faktor vergrößert wird und der sich danach in der Messkammer einstellende Gleichgewichtsdruck ermittelt wird und - basierend auf dem so erhaltenen Druckmesswert - der Gasgehalt der zu untersuchenden Flüssigkeit berechnet wird.

Der Erfindung liegt die Aufgabe zu Grunde, eine verbessertes Überwachungsverfahren zu schaffen, mit dem ermittelbar ist, wie gut die Abfüllung sauerstoffempfindlicher Produkte tatsächlich geklappt hat, um ggf. Optimierungspotentiale oder Fehlerquellen ausfindig zu machen.

### Zusammenfassung der Erfindung

Die Aufgabe wird gelöst durch die Merkmale des unabhängigen Anspruchs 1. Vorteilhafte Weiterbildungen sind in den abhängigen Ansprüchen und der Beschreibung angegeben.

Ein Aspekt der vorliegenden Offenbarung betrifft ein Verfahren zum Überwachen einer Behälterbehandlungsanlage, die eine Füllvorrichtung (z. B. ein Füllerkarussell) zum Befüllen von Behältern, vorzugsweise Dosen, und eine Verschließvorrichtung (z. B. Verschließerkarussell) zum Verschließen von Behältern, vorzugsweise Dosen, aufweist. Die Verschließvorrichtung ist (behälter-) stromabwärts von der Füllvorrichtung angeordnet (z. B. verbunden über eine Transportvorrichtung zwischen der Füllvorrichtung und der Verschließvorrichtung). Das Verfahren weist ein Füllen eines Behälters mit einem (z. B. flüssigem oder pastösem) Füllgut mittels der Füllvorrichtung auf. Das Verfahren weist ferner ein Messen einer Konzentration eines Gases in dem Füllgut des gefüllten Behälters mittels eines Messgeräts auf, wobei der gefüllte Behälter zum Messen der Konzentration stromaufwärts von der Verschließvorrichtung entnommen wird oder die Konzentration stromaufwärts von der Verschließvorrichtung gemessen wird.

Vorteilhaft ermöglicht das Verfahren, dass zielgerichtet die sauerstoffarme Abfüllung durch die Füllvorrichtung überwacht werden kann. Das Verfahren ist besonders geeignet für als Dosen ausgeführte Behälter, kann jedoch auch bei allen anderen Arten von Behältern eingesetzt werden. Zusätzliche Störfaktoren bzw. "Beunruhigungsquellen" für die Behälter, wie die Verschließvorrichtung, die die Behälter mit enormer Geschwindigkeit bewegen und behandeln und damit auch schütteln kann, sodass bspw. Kohlenstoffdioxid aus dem Füllgut im Behälter entweicht oder Sauerstoff vom Füllgut im Behälter aufgenommen wird, können das Messergebnis somit nicht verfälschen. Würde bspw. die Messung erst nach der Verschließvorrichtung stattfinden, so würde der Einfluss der Verschließvorrichtung die Konzentration des Gases im Füllgut des Behälters erheblich beeinflussen, sodass letztlich kaum eine sichere Aussage über die Qualität der Abfüllung durch die Füllvorrichtung getroffen werden könnte. Ursachen für Probleme beim Abfüllen lassen sich demzufolge durch das Verfahren gemäß der vorliegenden Offenbarung schneller identifizieren und beheben. Durch die zielgerichtete Analyse der Füllvorrichtung können bspw. monatelange Montageeinsätze zum Optimieren der Füllvorrichtung eingespart werden, wenn bspw. tatsächlich die Verschließvorrichtung für eine zu hohe Sauerstoffkonzentration oder zu geringe Kohlenstoffdioxidkonzentration im Füllgut in den Behältern verantwortlich ist. Damit kann auch ein Diskussionspotential bezüglich der Qualität der Füllvorrichtung zwischen Hersteller und Kunde der Füllvorrichtung verringert werden.

In einem Ausführungsbeispiel ist das Gas Kohlenstoffdioxid oder Sauerstoff.

Vorzugsweise kann sich der Ausdruck "Konzentration eines Gases im Füllgut" auf eine Angabe beziehen, die eine Masse (z. B. in g) oder ein Volumen (z. B. in I oder cm3) des Gases pro Masse (z. B. in g) oder Volumen (z. B. in I) des flüssigen oder pastösen Füllguts angibt. Unter diesem Ausdruck kann im Rahmen der vorliegenden Offenbarung auch ein Gasgehalt des Gases im Füllgut verstanden werden.

In einem weiteren Ausführungsbeispiel wird der gefüllte Behälter zum Ermitteln der Konzentration stromaufwärts von der Verschließvorrichtung manuell entnommen (z. B. ohne den gefüllten Behälter zu schütteln).

In einem weiteren Ausführungsbeispiel weist das Verfahren ein Abdecken, vorzugsweise manuelles Abdecken, des gefüllten Behälters mit einem Deckel, vorzugsweise im Wesentlichen gasdicht, vor dem Messen der Konzentration des Gases auf. Durch den Deckel kann der Behälter sicher zu dem Messgerät transportiert werden, auch wenn der Behälter wegen der Entnahme des Behälters stromaufwärts von der Verschließvorrichtung selbst noch gar nicht verschlossen wurde. Auch kann eine Veränderung der Atomsphäre im Kopfraum des gefüllten Behälters verhindert werden, die einen Einfluss auf den Gaswechsel zwischen Kopfraum und Füllgut im Behälter hat.

In einem weiteren Ausführungsbeispiel wird der gefüllte Behälter im Stillstand, ohne dass die Konzentration des Gases im Füllgut wesentlich verändert wird und/oder ohne Schütteln des gefüllten Behälters mit dem Deckel abgedeckt. Vorteilhaft können damit weitere Störfaktoren bzw. Beunruhigungsquellen, die die Gaskonzentration im Behälter beeinflussen können, ausgeschlossen werden.

In einer Ausführungsform wird der gefüllte Behälter direkt stromabwärts der Füllvorrichtung und/oder stromaufwärts von der Verschließvorrichtung mit dem Deckel abgedeckt. Damit kann der Behälter vorteilhaft ggf. noch vor oder direkt bei der Entnahme mit dem Deckel abgedeckt werden.

In einer weiteren Ausführungsform ist der Deckel gleich zu denjenigen Deckeln, mit dem die Verschließvorrichtung die Behälter, vorzugsweise Flaschen, verschließt. Alternativ kann der Deckel bspw. ein Adapterdeckel sein, der sich vorzugsweise von denjenigen Deckeln unterscheidet, mit dem die Verschließvorrichtung die Behälter, vorzugsweise Dosen, verschließt. Vorteilhaft kann die Technik somit sowohl bei Flaschen, z. B. PET-Flaschen oder Glasflaschen, die bspw. einen Schraubverschluss verwenden, und bei Dosen mit einem entsprechenden Adapterdeckel angewendet werden.

In einer weiteren Ausführungsform weist der Adapterdeckel eine, vorzugsweise durchstechbare, Öffnung auf, durch die hindurch das Füllgut zu dem Messgerät gefördert wird und/oder an der das Messgerät angeschlossen oder anschließbar ist.

In einer Ausführungsvariante weist der Adapterdeckel eine Dichtung, vorzugsweise O-Ring, zum Abdichten zu dem gefüllten Behälter (z. B. zu einem Außenmantel oder Innenmantel des Behälters) auf, vorzugsweise an einem umlaufenden Kragenabschnitt des Adapterdeckels.

In einer weiteren Ausführungsvariante weist das Messen der Konzentration des Gases ein Durchstechen des Deckels mittels einer Anstechvorrichtung und/oder ein Eintauchen eines Leitungsabschnitts, an den das Messgerät angeschlossen oder anschließbar ist, in das Füllgut im gefüllten Behälter auf.

Es ist möglich, dass der Leitungsabschnitt ein Teil der Anstechvorrichtung ist, z. B. derjenige Teil, mit dem der Deckel durchstochen wird (z. B. im Bereich der Öffnung des Deckels).

In einer weiteren Ausführungsvariante weist das Messen der Konzentration des Gases ein Herausdrücken und/oder Absaugen des Füllguts aus dem gefüllten Behälter hin zu dem Messgerät auf.

In einem Ausführungsbeispiel wird ein Fördergas, vorzugsweise Kohlenstoffdioxid oder Stickstoff, in den gefüllten Behälter, vorzugsweise in einen Kopfraum des gefüllten Behälters, eingeleitet, sodass das Füllgut aus dem gefüllten Behälter hin zu dem Messgerät gedrückt wird. Vorteilhaft kann damit auf einfache Weise eine Förderung des Füllguts hin zu dem Messgerät ermöglicht werden, ohne dass dabei das Füllgut durch Sauerstoff im Fördergas verunreinigt werden würde.

In einem Ausführungsbeispiel wird ein Spülgas, vorzugsweise Kohlenstoffdioxid oder Stickstoff, zum Spülen einer Leitung zwischen dem gefüllten Behälter und dem Messgerät in die Leitung eingeleitet, bevor das Füllgut durch die Leitung zu dem Messgerät geleitet wird. Vorteilhaft kann damit verhindert werden, dass das Füllgut auf dem Weg zum Messgerät durch Sauerstoff in der Leitung verunreinigt wird, wodurch das Messergebnis verfälschen werden könnte.

In einem weiteren Ausführungsbeispiel weist das Verfahren ferner ein Anpassen eines Betriebs und/oder einer Konfiguration (z. B. Aufbau, Montage und/oder Steuerung) der Füllvorrichtung in Abhängigkeit von der gemessenen Konzentration auf. Vorteilhaft kann damit auf Basis der gemessenen Konzentration, die nicht durch stromabwärts der Füllvorrichtung liegende Störfaktoren beeinflusst wurde, sehr zielgerichtet erkannt werden, ob der Betrieb oder die Konfiguration der Füllvorrichtung anzupassen (z. B. zu optimieren) ist, um prozesssicher die gewünschte Konzentration des Gases im Füllgut im Behälter direkt stromabwärts der Füllvorrichtung zu erhalten.

In einem weiteren Ausführungsbeispiel weist das Verfahren ferner ein Messen einer weiteren Konzentration des Gases in dem Füllgut, bevor das Füllgut in den Behälter gefüllt wird und/oder in einem Füllgutzulauf zu der Füllvorrichtung auf, wobei vorzugsweise das Füllgut zum Messen der weiteren Konzentration des Gases an einem Zapfventil des Füllgutzulaufs abgezapft wird. Optional kann das Verfahren ferner ein Ermitteln einer Konzentrationsveränderung des Gases über die Füllvorrichtung in Abhängigkeit von der gemessenen Konzentration und der gemessenen weiteren Konzentration aufweisen. Vorteilhaft kann damit eine Änderung der Konzentration des Gases, die tatsächlich durch die Behandlung in der Füllvorrichtung bewirkt ist, abgeleitet werden, um ggf. Optimierungspotentiale bei der Füllvorrichtung zu identifizieren oder um zu erkennen, dass die Füllvorrichtung bereits wie gewünscht bzw. optimal eingestellt ist.

In einem weiteren Ausführungsbeispiel erfolgt das Anpassen des Betriebs und/oder der Konfiguration (z. B. Aufbau, Montage und/oder Steuerung) der Füllvorrichtung ferner in Abhängigkeit von der gemessenen weiteren Konzentration. Bevorzugt kann das Anpassen (z. B. nur) unter der Bedingung erfolgen, dass die Konzentrationsveränderung des Gases über die Füllvorrichtung größer oder kleiner als ein vorgegebener Grenzwert ist.

Bevorzugt kann Behälterbehandlungsanlage zum Herstellen, Reinigen, Beschichten, Prüfen, Abfüllen, Verschließen, Etikettieren, Bedrucken und/oder Verpacken von Behältern für flüssige Medien, vorzugsweise Getränke oder flüssige Nahrungsmittel, ausgeführt sein.

Beispielsweise können die Behälter als Flaschen, Dosen, Kanister, Kartons, Flakons usw. ausgeführt sein.

Die zuvor beschriebenen bevorzugten Ausführungsformen und Merkmale der Erfindung sind beliebig miteinander kombinierbar.

### Kurzbeschreibung der Figuren

Weitere Einzelheiten und Vorteile der Erfindung werden im Folgenden unter Bezug auf die beigefügten Zeichnungen beschrieben. Es zeigen:
- Figur 1: eine schematische Ansicht einer Behälterbehandlungsanlage;
- Figur 2: ein schematischer Aufbau zum Messen einer Konzentration eines Gases in einem Füllgut eines Behälters, wobei der Behälter und ein Adapterdeckel im Schnitt dargestellt sind; und
- Figur 3: ein Säulendiagramm zum Aufzeigen von beispielhaft gemessenen Konzentrationen von Kohlenstoffdioxid im Füllmedium an unterschiedlichen Positionen einer Behälterbehandlungsanlage.

### Detaillierte Beschreibung beispielhafter Ausführungsformen

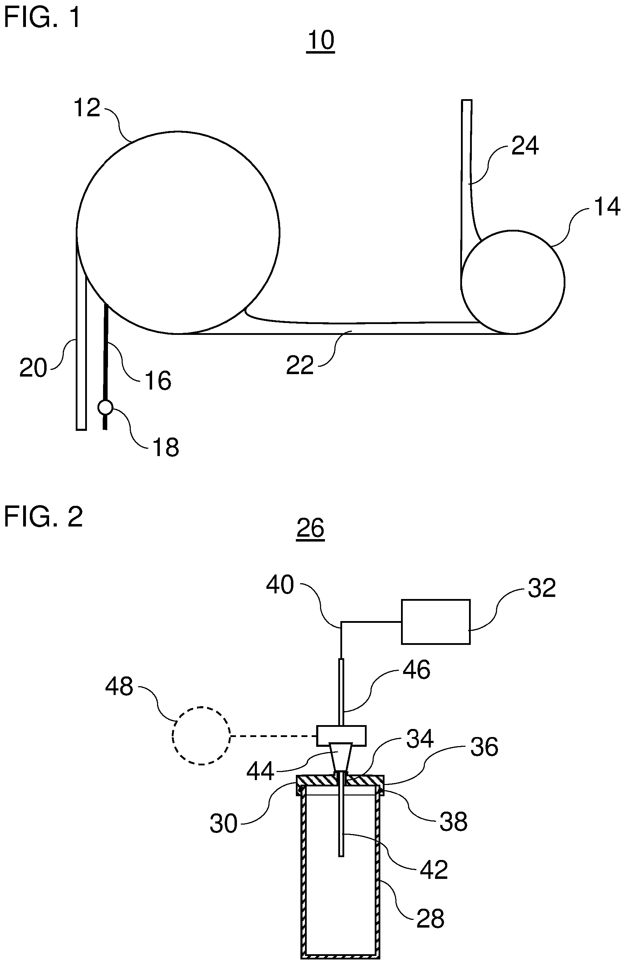

Die Figur 1 zeigt eine Behälterbehandlungsanlage 10. Die Behälterbehandlungsanlage 10 weist eine Füllvorrichtung 12 zum Füllen von Behältern und eine Verschließvorrichtung 14 zum Verschließen von Behältern auf. Die Verschließvorrichtung 14 ist (behälter-) stromabwärts von der Füllvorrichtung 12 angeordnet.

Besonders bevorzugt ist die Behälterbehandlungsanlage zum Behandeln von Dosen, wie Getränkedosen (z. B. 0,33 I- oder 0,5 I-Dosen), ausgebildet. Dementsprechend ist die Füllvorrichtung 12 bevorzugt zum Füllen von Dosen ausgeführt, und die Verschließvorrichtung 14 ist bevorzugt zum Verschließen von Dosen ausgeführt.

Es ist allerdings prinzipiell auch möglich, die Techniken der vorliegenden Offenbarung auf Behälterbehandlungsanlagen anzuwenden, die eine andere Art von Behältern behandeln können. Derartige Behälter können beispielsweise als Flaschen, Kanister, Kartons, Flakons usw. ausgeführt sein.

Der Füllvorrichtung 12 ist bevorzugt als ein Füllerkarussell ausgeführt. Die Verschließvorrichtung 14 ist bevorzugt als ein Verschließerkarussell ausgeführt. Es sind allerdings auch andere Bauformen für die Füllvorrichtung 12 und/oder die Verschließvorrichtung 14 denkbar, zum Beispiel eine Ausführung als Linearfüller bzw. als ein Linearverschließer.

Die Füllvorrichtung 12 kann die Behälter mit einem Füllgut, zum Beispiel einem Getränk oder einem Nahrungsmittel, füllen. Das Füllgut ist vorzugsweise ein flüssiges oder pastöses Medium. Besonders bevorzugt ist das Füllgut sauerstoffempfindlich, wie beispielsweise Bier.

Die Füllvorrichtung 12 kann mehrere Füllventile zum gleichzeitigen Befüllen mehrerer Behälter aufweisen. Bspw. können die Füllventile um einen Umfang der als Füllerkarussell ausgeführten Füllvorrichtung 12 angeordnet sein.

Die Füllvorrichtung 12 kann einen Produkt- bzw. Füllgutzulauf 16 aufweisen. Über den Füllgutzulauf 16 kann der Füllvorrichtung 12 (und damit den Füllventilen der Füllvorrichtung 12) das Füllgut zugeführt werden. Es ist möglich, dass der Füllgutzulauf 16 beispielsweise ein Zapfventil 18, zum Beispiel einen Produkthahn, aufweist. An dem Zapfventil 18 kann das Füllgut bspw. für Untersuchungen abgezapft werden, z. B. manuell oder automatisch.

Die Füllvorrichtung 12 kann einen Behälterzulauf 20 aufweisen. Über den Behälterzulauf 20 können Behälter zu der Füllvorrichtung 12 zum Füllen zugeführt werden. Der Behälterzulauf 20 kann beispielsweise einen oder mehrere Behälterförderer aufweisen. Der oder die Behälterförderer können beispielsweise mindestens einen Transportstern (z. B. Füllvorrichtung-Einlaufstern) und/oder Linearförderer aufweisen.

Stromabwärts von der Füllvorrichtung 12 kann eine Transportvorrichtung 22 der Behälterbehandlungsanlage 10 angeordnet sein. Die Transportvorrichtung 22 kann die Füllvorrichtung 12 und die Verschließvorrichtung 14 miteinander verbinden. Die Transportvorrichtung 22 kann zum Transportieren von Behältern von der Füllvorrichtung 12 zu der Verschließvorrichtung 14 ausgeführt sein. Die Transportvorrichtung 22 kann einen oder mehrere Behälterförderer aufweisen. Der oder die Behälterförderer können beispielsweise mindestens einen Transportstern (z. B. Füllvorrichtung-Auslaufstern und/oder Verschließvorrichtung-Einlaufstern) und/oder Linearförderer aufweisen.

Die Verschließvorrichtung 14 kann die Behälter verschließen. Bevorzugt kann die Verschließvorrichtung 14 die als Dosen ausgeführten Behälter mit einem Dosendeckel verschließen. Der Dosendeckel kann fest mit dem Behälter verbunden werden, zum Beispiel durch mechanisches Umformen des Dosendeckels und/oder des Behälters. Der Dosendeckel selbst kann wiederum einen öffenbaren Verschluss aufweisen, wie dies beispielsweise von Getränkedosen bekannt ist.

Es ist allerdings auch möglich, dass die Verschließvorrichtung 14 zum Verschließen einer anderen Art von Behältern, also nicht von Dosen, ausgeführt ist. Dementsprechend kann die Verschließvorrichtung 14 die Behälter beispielsweise mit einem Deckel, einem Korken, einem Kronkorken oder einem Schraubverschluss verschließen. Die Verschließvorrichtung 14 kann mehrere Verschließstationen zum gleichzeitigen Verschließen mehrerer Behälter aufweisen. Bspw. können die Verschließstationen um einen Umfang der als Verschließerkarussell ausgeführten Verschließvorrichtung 14 angeordnet sein.

Die von der Verschließvorrichtung 14 verschlossenen Behälter können die Verschließvorrichtung 14 über einen Behälterauslauf 24 verlassen. Der Behälterauslauf 24 kann die verschlossenen Behälter beispielsweise zu mindestens einer weiteren Behandlungsvorrichtung der Behälterbehandlungsanlage transportieren. Der Behälterauslauf 24 kann einen oder mehrere Behälterförderer aufweisen. Der oder die Behälterförderer können beispielsweise mindestens einen Transportstern (z. B. Füllvorrichtung-Auslaufstern und/oder Verschließvorrichtung-Einlaufstern) und/oder Linearförderer aufweisen.

Es ist möglich, dass die Behälterbehandlungsanlage 10 zusätzlich zu der Füllvorrichtung 12 und der Verschließvorrichtung 14 weitere Behandlungsvorrichtungen zum Behandeln von Behältern aufweist (nicht dargestellt). Beispielsweise kann die Behälterbehandlungsanlage 10 zusätzlich eine Behandlungsvorrichtung zum Herstellen, Prüfen, Beschichten, Reinigen, Etikettieren, Bedrucken und/oder Verpacken von Behältern aufweisen.

Die Figur 2 zeigt einen beispielhaften Messaufbau 26 zum Messen einer Konzentration eines Gases im Füllmedium eines Behälters 28.

Der Messaufbau 26 weist den Behälter 28, einen Adapterdeckel 30 und ein Messgerät 32 auf. Der Behälter 28 ist bevorzugt als eine unvollständige Dose ausgeführt, bei der der herkömmliche Dosenverschluss fehlt, wie dargestellt ist.

Der Adapterdeckel 30 ist bevorzugt dazu ausgebildet, den als unvollständige Dose ausgeführten Behälter 28, bei dem der herkömmliche Dosenverschluss / Deckel fehlt, zu verschließen. Statt eines herkömmlichen Verschlusses / Deckels kann der Behälter 28 somit mittels des Adapterdeckels 30 verschlossen werden.

Bevorzugt weist der Adapterdeckel 30 eine Öffnung 34 auf. Beispielsweise kann die Öffnung 34 zentral im Adapterdeckel 30 angeordnet sein. Die Öffnung 34 kann bevorzugt eine Oberseite des Adapterdeckels 30 mit einer Unterseite des Adapterdeckels 30 verbinden. Die Öffnung 34 kann als ein Durchgangsloch durch den Adapterdeckel 30 ausgeführt sein.

Es ist möglich, dass die Öffnung 34 Teil bzw. Abschnitt eines Befestigungsanschlusses zum, vorzugsweise gasdichten, lösbaren Anschließen des Messgeräts 32 ist. Der Befestigungsanschluss kann bspw. einen Befestigungsabschnitt, wie bspw. ein Gewinde, ein Bajonettverschluss, ein Steckelement, eine Steckelementaufnahme, ein Rastelement oder eine Rastelementaufnahme zum Anschließen des Messgeräts 32 aufweisen.

Der Adapterdeckel 30 kann bevorzugt scheibenförmig ausgeführt sein und einen umlaufenden Kragenabschnitt bzw. Bund 36 aufweisen. Zum Verschließen des Behälters 28, kann der Kragenabschnitt 36 einen Außenmantel des Behälters 28 vollumfänglich umgreifen bzw. umgeben. Am Kragenabschnitt 36 kann ferner eine Dichtung 38, wie bspw. ein O-Ring, angeordnet sein. Die Dichtung 38 kann zwischen dem Adapterdeckel 30 bzw. dessen Kragenabschnitt 36 und dem Behälter 28 bzw. dessen Außenmantel gasdicht abdichten.

Eine Verbindung zwischen dem Adapterdeckel 30 und dem Behälter 28 kann lösbar sein. Bevorzugt kann der Adapterdeckel 30 auf den unverschlossenen Behälter 28 aufgesteckt bzw. aufgesetzt werden.

Das Messgerät 32 kann an dem Adapterdeckel 30 (oder einem anderen Deckel) zum Messen einer Konzentration eines Gases, vorzugsweise Kohlenstoffdioxid (CO2) oder Sauerstoff, lösbar angeschlossen werden. Das Messgerät 32 kann über eine Leitung 40 an dem Adapterdeckel 30 und damit dem Behälter 28 angeschlossen sein.

Das Messgerät 32 kann jegliche bekannte Technik nutzen, um eine Konzentration des Gases zu messen. Das Messgerät 32 kann die gemessene Konzentration zudem ausgeben, z. B. über eine Anzeigeeinheit oder in Form eines entsprechenden elektrischen (z. B. analogen oder digitalen) Signals, das die gemessene Konzentration angibt.

Das Messgerät 32 kann über eine Dichtung 44 an dem Adapterdeckel 30 (oder einem anderen Deckel), bevorzugt an dessen Öffnung 34, angeschlossen sein. Beispielsweise kann die Dichtung 44 eine Keilstumpf- oder Kegelstumpfform zum Einschieben in die Öffnung 34 aufweisen.

Bevorzugt ragt ein Leitungsabschnitt 42, der mit dem Messgerät 32 verbunden ist, durch die Öffnung 34 direkt in das Füllmedium im Behälter 28. Über diesen Leitungsabschnitt 42 kann zumindest ein Teil des Füllmediums im Behälter 28 zu dem Messgerät 32 geleitet werden. In diesem Teil des Füllmediums aus dem Behälter 28 kann das Messgerät 32 die Konzentration des Gases messen.

Es ist möglich, dass der Messaufbau 26 ferner eine Anstechvorrichtung 46 und/oder eine Gasquelle 48 aufweist.

Die Anstechvorrichtung 46 kann dazu ausgebildet sein, den Adapterdeckel 30 (oder einem anderen Deckel) im Bereich der ggf. verschlossenen Öffnung 34 anzustechen oder durch die Öffnung 34 hindurchgeführt zu werden, um mit dem Leitungsabschnitt 42 in das Füllmedium im Behälter 28 einzutauchen.

Die Gasquelle 48 kann mit einem Leitungssystem, das das Messgerät 32 und die Öffnung 34 des Adapterdeckels 30 miteinander verbindet, verbunden sein. Die Gasquelle 48 kann bspw. Stickstoff oder Kohlenstoffdioxid bereitstellen. Mittels der Gasquelle 48 kann Gas in das Leitungssystem geleitet werden. Das Gas kann bspw. als Spülgas zum Spülen des Leitungssystems verwendet werden. Alternativ oder zusätzlich kann das Gas als Fördergas zum Herausdrücken des Füllmediums im Behälter 28 durch den Leitungsabschnitt 42 hin zu dem Messgerät 32 verwendet werden. Hierfür kann das Gas bspw. in einen Kopfraum des Behälters 28 zugeführt werden.

Auch wenn hierin das besonders bevorzugte Ausführungsbeispiel mit dem als Dose ausgeführten Behälter 28 und dem Adapterdeckel 30 beschrieben ist, wird darauf hingewiesen, dass in Ausführungsformen, in denen die Behälter 28 bspw. keine Dosen sind (z. B. Glas- oder PET-Flaschen), statt des Adapterdeckels 30 ein anderer Deckel zum Verschließen des Behälters 28 zum Anschließen des Messgeräts 32 verwendet werden kann. Der Deckel kann bevorzugt vom gleichen Typ sein wie von der Verschließvorrichtung 14 zum Verschließen der Behälter 28 verwendet, z. B. ein Schraubverschluss.

Nachfolgend ist ein Verfahren zum Überwachen einer Behälterbehandlungsanlage beschrieben. Das Verfahren ist beispielhaft anhand der Figuren 1 bis 3 beschrieben.

Das Verfahren weist ein Füllen eines Behälters 28 mit einem Füllgut mittels der Füllvorrichtung 12 auf. Beispielsweise kann der Behälter 28 als einer von mehreren Behältern 28 von der Füllvorrichtung 12 mit dem Füllmedium gefüllt werden. Beim Füllen kann der Behälter 28 unterhalb von einem Füllventil der Füllvorrichtung 12 positioniert sein. Beim Füllen kann der Behälter 28 an das Füllventil angepresst sein, oder es kann ein Spalt zwischen dem Behälter 28 und dem Füllventil vorhanden sein. Die Füllvorrichtung 12 kann das Füllmedium über den Füllgutzulauf 16 empfangen. Die Füllvorrichtung 12 kann den zu füllenden Behälter 28 über den Behälterzulauf 20 empfangen.

Es ist möglich, dass der Behälter 28 vor dem Füllen mit dem Füllmedium mindestens einmal evakuiert, mindestens einmal mit einem Produktionsgas (z. B. Inertgas, wie Kohlenstoffdioxid) gespült und/oder mit einem Produktionsgas (z. B. Inertgas, wie Kohlenstoffdioxid) vorgespannt wird. Das Evakuieren, Spülen und/oder Vorspannen kann mittels der Füllvorrichtung 12 erfolgen.

Das Verfahren weist ferner ein Messen einer Konzentration eines Gases in dem Füllmedium des gefüllten Behälters 28 mittels des Messgeräts 32 auf. Dafür kann das Füllgut aus dem Behälter 28 zu dem Messgerät 32 geleitet werden.

Der mittels der Füllvorrichtung 12 mit dem Füllmedium gefüllte Behälter 28 wird zum Messen der Konzentration stromaufwärts von der Verschließvorrichtung 14 entnommen, z. B. direkt stromabwärts von der Füllvorrichtung 10 und/oder von einer Position auf der Transportvorrichtung 22. Beim Entnehmen kann die Transportvorrichtung 22 zum Transportieren von Behältern 28 in Bewegung sein oder stillstehen.

Alternativ wird die Konzentration des Gases im Füllmedium des gefüllten Behälters 28 stromaufwärts von der Verschließvorrichtung 14 gemessen, ohne dass der gefüllte Behälter 28 dafür von einer Position auf der Transportvorrichtung 22 entnommen wird. In diesem Fall kann die Konzentration des Gases im Füllmedium bspw. direkt an der Transportvorrichtung 22 gemessen werden.

Das Entnehmen des gefüllten Behälters 28 kann bevorzugt manuell / händisch erfolgen. Es ist allerdings auch möglich, dass der gefüllte Behälter 28 bspw. automatisch aus eine Behälterstrom ausgeleitet und damit entnommen wird.

Das Verfahren kann ferner ein Abdecken, vorzugsweise manuelles Abdecken, des gefüllten Behälters 28 mit einem (z. B. Adapter-) Deckel 30 vor dem Messen der Konzentration des Gases aufweisen. Der Deckel 30 kann den Behälter 28 bevorzugt gasdicht abdecken. Der Deckel 30 kann bspw. wie der Adapterdeckel 30 (siehe Figur 2) ausgeführt sein, insbesondere wenn der Behälter 28 eine Dose ist.

Das Abdecken mit dem Deckel 30 erfolgt bevorzugt im Stillstand des Behälters 28, z. B. wenn der Behälter 28 von der Transportvorrichtung 22 gehalten oder gestützt ist, und die Transportvorrichtung 22 stillsteht. Bevorzugt kann der gefüllte Behälter 28 direkt stromabwärts der Füllvorrichtung 12 mit dem Deckel 30 abgedeckt werden.

Der Behälter 28 kann jedoch bspw. auch von der Transportvorrichtung 22 entnommen, woanders abgestellt und dann mit dem Deckel 30 abgedeckt werden. Alternativ kann der Deckel 30 auch während einer Bewegung des Behälters 28 mit dem Deckel 30 abgedeckt werden, wobei es dabei bevorzugt zu keinem Schütteln des Behälters 28 und damit zu keiner wesentlichen Änderung der Konzentration des Gases im Füllgut im Behälter 28 kommt.

Das Messen bzw. Ermitteln der Konzentration des Gases mittels des Messgeräts 32 kann ein Anschließen des Messgeräts 32 an dem Deckel 30, z. B. der Öffnung 34 des Deckels 30 aufweisen. Hierbei kann bspw. die Anstechvorrichtung 46 verwendet werden, um den Deckel 30 anzustechen. Der Leitungsabschnitt 42 kann in das Füllgut im Behälter 28 eintauchen und das Füllgut zu dem Messgerät 32 leiten.

Es ist möglich, dass das Füllgut aus dem Behälter 28 zu dem Messgerät 32 abgesaugt wird, z. B. mit einer Pumpe (nicht dargestellt). Alternativ oder zusätzlich kann das Füllgut aus dem Behälter 28 durch Zuführen eines (z. B. Förder-) Gases in den Kopfraum des Behälters 28 aus dem Behälter 28 über den Leistungsabschnitt 42 in Richtung zu dem Messgerät 32 herausgedrückt werden. Das Gas kann von Gasquelle 48 zu dem Behälter 28 geleitet werden. Es ist auch möglich, dass das Gas von der Gasquelle 48 die Leitung zwischen dem Behälter 28 und dem Messgerät 32 spült.

Das Verfahren kann ferner ein Anpassen eines Betriebs und/oder einer Konfiguration der Füllvorrichtung 12 in Abhängigkeit von der gemessenen Konzentration aufweisen.

Beispielsweise kann eine Sauerstoffkonzentration gemessen werden, die über einem vorgegebenen Grenzwert liegt. Der Betrieb und/oder die Konfiguration der Füllvorrichtung 12 kann angepasst werden, um die Sauerstoffkonzentration im Füllgut in nachfolgend abgefüllten Behältern 28 zu verringern.

Andererseits kann bspw. eine Kohlenstoffdioxidkonzentration gemessen werden, die unter einem vorgegebenen Grenzwert liegt. Der Betrieb und/oder die Konfiguration der Füllvorrichtung 12 kann angepasst werden, um die Kohlenstoffdioxidkonzentration im Füllgut in nachfolgend abgefüllten Behältern 28 zu erhöhen.

Das Verfahren kann ferner ein Messen einer (weiteren) Konzentration des Gases in dem Füllgut aufweisen, und das Anpassen des Betriebs und/oder der Konfiguration der Füllvorrichtung 12 kann ferner abhängig von der gemessenen weiteren Konzentration sein.

Diese (weitere) Konzentration des Gases kann im Füllgut gemessen werden, bevor das Füllgut von der Füllvorrichtung 12 in die Behälter 28 gefüllt wird. Beispielsweise kann Füllgut am Zapfventil 18 im Füllgutzulauf 16 abgezapft und auf die (weitere) Konzentration hin untersucht werden, z. B. mit dem Messgerät 32.

Aus der gemessenen Konzentration des Füllguts im Behälter 28 und der gemessenen (weiteren) Konzentration des Füllguts vor dem Abfüllen kann eine Konzentrationsveränderung des Gases im Füllgut über die Füllvorrichtung 12 abgeleitet bzw. ermittelt werden. Die Konzentration kann zugenommen, abgenommen oder im Wesentlichen gleichgeblieben sein.

Wenn bspw. eine Zunahme der Sauerstoffkonzentration über die Füllvorrichtung 12 ermittelt wurde, die einen vorgegebenen Grenzwert überschreitet, kann der Betrieb und/oder die Konfiguration der Füllvorrichtung 12 angepasst werden, um bei nachfolgend befüllten Behältern 28 eine geringe Sauerstoffkonzentration im Füllgut in den Behältern 28 zu erhalten.

Wenn bspw. eine Abnahme der Kohlenstoffdioxidkonzentration über die Füllvorrichtung 12 ermittelt wurde, die einen vorgegebenen Grenzwert überschreitet, kann der Betrieb und/oder die Konfiguration der Füllvorrichtung 12 angepasst werden, um bei nachfolgend befüllten Behältern 28 eine höhere Kohlenstoffdioxidkonzentration im Füllgut in den Behältern 28 zu erhalten.

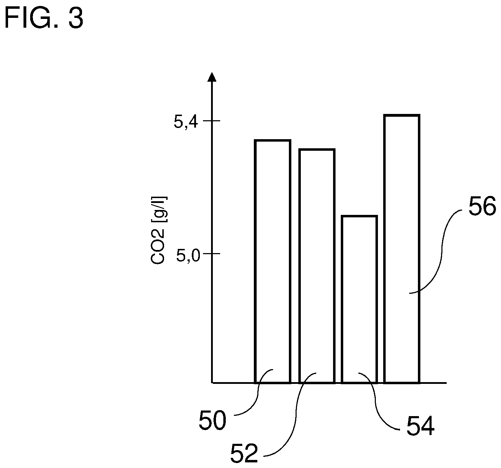

Das Verfahren ermöglicht damit eine sehr genaue Untersuchung dahingehend, inwiefern die Füllvorrichtung 12 für eine Zu- oder Abnahme der Konzentration des Gases verantwortlich ist, um entsprechend zielgerichtet reagieren zu können. Damit ist das Verfahren im Vorteil gegenüber anderen Verfahren, wie nachfolgend unter Bezugnahme auf die Figur 3 beschrieben ist.

Die Figur 3 zeigt ein Säulendiagramm mit vier Säulen 50, 52, 54, 56, die tatsächlich gemessene Kohlenstoffdioxidkonzentrationen in einer Behälterbehandlungsanlage für als Dosen ausgeführte Behälter 28 angeben.

Die Säule 50 gibt eine Kohlenstoffdioxidkonzentration im Füllgut an, die am Zapfventil 18 gemessen wurde. Die Säule 52 gibt eine Kohlenstoffdioxidkonzentration im Füllgut befüllter, verschlossener Behälter 28 an, die stromabwärts von der Verschließvorrichtung 14 gemessen wurde. Die Säule 54 gibt eine Kohlenstoffdioxidkonzentration im Füllgut befüllter Behälter 28 an, die gemessen wurde, nachdem der befüllte Behälter 28 kräftig geschüttelt wurde. Durch das Schütteln wird die Kohlenstoffdioxidkonzentration im Füllgut im befüllten Behälter 28 wesentlich verringert. Die Säule 56 gibt eine Kohlenstoffdioxidkonzentration im Füllgut befüllter Behälter 28 an, die gemäß dem vorliegenden Überwachungsverfahren stromaufwärts von der Verschließvorrichtung 14 gemessen wurde, z. B. nach entsprechender Entnahme des Behälter 28.

Beim Vergleich der Säulen 50 und 52 fällt eine Abnahme der Kohlenstoffdioxidkonzentration auf. Beim Vergleich der Säulen 50 und 56 fällt hingegen eine Zunahme der Kohlenstoffdioxidkonzentration auf. Die Dose, die nach der Verschließvorrichtung 14 zum Messen entnommen wurde, weist rd. 0,1 g/l CO2 weiniger im Füllgut auf als die Dose, die zwischen der Füllvorrichtung 12 und der Verschließvorrichtung 14 zum Messen entnommen wurde (siehe Säulen 52 vs. 56).

D.h., nur der Vergleich der Säulen 50 und 56 kann verlässlich darüber Auskunft geben, wie sich die Konzentration von Kohlenstoffdioxid über die Füllvorrichtung 12 verändert hat bzw. welcher Kohlenstoffdioxidgehalt im Füllgut im Behälter 28 direkt nach dem Befüllen herrscht. Würde man nur die Säulen 50 und 52 betrachten, käme man möglicherweise zu Fehleinschätzungen bzw. Fehldiagnosen. Es würde nicht berücksichtigt werden, dass aufgrund der Bewegungen und Beunruhigungen der Behälter 28 in der Verschließvorrichtung 14 die Behälter 28 geschüttelt werden und damit eine Kohlenstoffdioxidkonzentration im Füllgut in den Behältern 28 abnehmen kann. Man würde folglich die Qualität der (sauerstoffarmen) Abfüllung der Füllvorrichtung 12 unterschätzen. Nur über die Betrachtung der Säule 56 kann zielgerichtet auf die tatsächliche Aufnahme oder Abgabe von CO2 beim Befüllen geschlossen werden. Dieser lässt sich, wie erläutert, bei den Dosen mittels des Messaufbaus 26, der unter Bezugnahme auf die Figur 2 beschrieben ist, unter Verwendung des Adapterdeckels 30 für den als Dose ausgeführten Behälter 28 messen, bevor der Behälter 28 von der Verschließvorrichtung 14 verschlossen wurde.

Die Figur 3 bezieht sich beispielhaft auf die Messung von Kohlenstoffdioxidkonzentrationen. Die Technik ist jedoch bspw. auch bei der Messung von Sauerstoffkonzentrationen anwendbar.

Die Erfindung ist nicht auf die vorstehend beschriebenen bevorzugten Ausführungsbeispiele beschränkt. Vielmehr ist eine Vielzahl von Varianten und Abwandlungen möglich, die ebenfalls von dem Erfindungsgedanken Gebrauch machen und deshalb in den Schutzbereich fallen. Insbesondere beansprucht die Erfindung auch Schutz für den Gegenstand und die Merkmale der Unteransprüche unabhängig von den in Bezug genommenen Ansprüchen. Insbesondere sind die einzelnen Merkmale des unabhängigen Anspruchs 1 jeweils unabhängig voneinander offenbart. Zusätzlich sind auch die Merkmale der Unteransprüche unabhängig von sämtlichen Merkmalen des unabhängigen Anspruchs 1 und beispielsweise unabhängig von den Merkmalen des unabhängigen Anspruchs 1 offenbart.

### Bezugszeichenliste

- 10: Behälterbehandlungsanlage
- 12: Füllvorrichtung
- 14: Verschließvorrichtung
- 16: Füllgutzulauf
- 18: Zapfventil
- 20: Behälterzulauf
- 22: Transportvorrichtung
- 24: Behälterauslauf
- 26: Messaufbau
- 28: Behälter
- 30: Adapterdeckel
- 32: Messgerät
- 34: Öffnung
- 36: Kragenabschnitt
- 38: Dichtung
- 40: Leitung
- 42: Leitungsabschnitt
- 44: Dichtung
- 46: Anstechvorrichtung
- 48: Gasquelle
- 50-56: Säulen zur Angabe der Kohlenstoffdioxidkonzentration

## Patentansprüche

1. Verfahren zum Überwachen einer Behälterbehandlungsanlage (10), die eine Füllvorrichtung (12) zum Befüllen von Behältern (28), vorzugsweise Dosen, und eine Verschließvorrichtung (14) zum Verschließen von Behältern (28), vorzugsweise Dosen, aufweist, wobei die Verschließvorrichtung (14) stromabwärts von der Füllvorrichtung (12) angeordnet ist und das Verfahren aufweist:
Füllen eines Behälters (28) mit einem Füllgut mittels der Füllvorrichtung (12);
Messen einer Konzentration eines Gases in dem Füllgut des gefüllten Behälters (28) mittels eines Messgeräts (32), wobei der gefüllte Behälter (28) zum Messen der Konzentration stromaufwärts von der Verschließvorrichtung (14) entnommen wird oder die Konzentration stromaufwärts von der Verschließvorrichtung (14) gemessen wird.

2. Verfahren nach Anspruch 1, wobei:
das Gas Kohlenstoffdioxid oder Sauerstoff ist.

3. Verfahren nach Anspruch 1 oder Anspruch 2, wobei:
der gefüllte Behälter (28) zum Ermitteln der Konzentration stromaufwärts von der Verschließvorrichtung (14) manuell entnommen wird.

4. Verfahren nach einem der vorherigen Ansprüche, ferner aufweisend:
Abdecken, vorzugsweise manuelles Abdecken, des gefüllten Behälters (28) mit einem Deckel (30), vorzugsweise im Wesentlichen gasdicht, vor dem Messen der Konzentration des Gases.

5. Verfahren nach Anspruch 4, wobei:
wobei der gefüllte Behälter (28) im Stillstand, ohne dass die Konzentration des Gases im Füllgut wesentlich verändert wird und/oder ohne Schütteln des gefüllten Behälters (28) mit dem Deckel (30) abgedeckt wird.

6. Verfahren nach Anspruch 4 oder Anspruch 5, wobei:
wobei der gefüllte Behälter (28) direkt stromabwärts der Füllvorrichtung (12) und/oder stromaufwärts von der Verschließvorrichtung (14) mit dem Deckel (30) abgedeckt wird.

7. Verfahren nach einem der Ansprüche 4 bis 6, wobei:
der Deckel (30) gleich zu denjenigen Deckeln ist, mit dem die Verschließvorrichtung (14) die Behälter (28), vorzugsweise Flaschen, verschließt, oder
der Deckel (30) ein Adapterdeckel ist, der sich vorzugsweise von denjenigen Deckeln unterscheidet, mit dem die Verschließvorrichtung (14) die Behälter (28), vorzugsweise Dosen, verschließt.

8. Verfahren nach Anspruch 7, wobei:
der Adapterdeckel (30) eine, vorzugsweise durchstechbare, Öffnung (34) aufweist, durch die hindurch das Füllgut zu dem Messgerät (32) gefördert wird und/oder an der das Messgerät (32) angeschlossen oder anschließbar ist.

9. Verfahren nach Anspruch 7 oder Anspruch 8, wobei:
der Adapterdeckel (30) eine Dichtung (38), vorzugsweise O-Ring, zum Abdichten zu dem gefüllten Behälter (28) aufweist, vorzugsweise an einem umlaufenden Kragenabschnitt (36) des Adapterdeckels (30).

10. Verfahren nach einem der Ansprüche 5 bis 9, wobei:
das Messen der Konzentration des Gases ein Durchstechen des Deckels (30) mittels einer Anstechvorrichtung (46) und/oder ein Eintauchen eines Leitungsabschnitts (42), an der das Messgerät (32) angeschlossen oder anschließbar ist, in das Füllgut im gefüllten Behälter (28) aufweist.

11. Verfahren nach einem der vorherigen Ansprüche, wobei:
das Messen der Konzentration des Gases ein Herausdrücken und/oder Absaugen des Füllguts aus dem gefüllten Behälter (28) hin zu dem Messgerät (32) aufweist.

12. Verfahren nach einem der vorherigen Ansprüche, wobei:
ein Fördergas, vorzugsweise Kohlenstoffdioxid oder Stickstoff, in den gefüllten Behälter (28), vorzugsweise in einen Kopfraum des gefüllten Behälters (28), eingeleitet wird, sodass das Füllgut aus dem gefüllten Behälter (28) hin zu dem Messgerät (32) gedrückt wird, und/oder
ein Spülgas, vorzugsweise Kohlenstoffdioxid oder Stickstoff, zum Spülen einer Leitung (40) zwischen dem gefüllten Behälter (28) und dem Messgerät (32) in die Leitung (40) eingeleitet wird, bevor das Füllgut durch die Leitung zu dem Messgerät (32) geleitet wird.

13. Verfahren nach einem der vorherigen Ansprüche, ferner aufweisend:
Anpassen eines Betriebs und/oder einer Konfiguration der Füllvorrichtung (12) in Abhängigkeit von der gemessenen Konzentration.

14. Verfahren nach einem der vorherigen Ansprüche, ferner aufweisend:
Messen einer weiteren Konzentration des Gases in dem Füllgut, bevor das Füllgut in den Behälter (28) gefüllt wird und/oder in einem Füllgutzulauf (16) zu der Füllvorrichtung (12), wobei vorzugsweise das Füllgut zum Messen der weiteren Konzentration des Gases an einem Zapfventil (18) des Füllgutzulaufs (16) abgezapft wird, und optional
Ermitteln einer Konzentrationsveränderung des Gases über die Füllvorrichtung (12) in Abhängigkeit von der gemessenen Konzentration und der gemessenen weiteren Konzentration.

15. Verfahren nach Anspruch 13 und Anspruch 14, wobei:
das Anpassen des Betriebs und/oder der Konfiguration der Füllvorrichtung (12) ferner in Abhängigkeit von der gemessenen weiteren Konzentration erfolgt, und
das Anpassen vorzugsweise unter der Bedingung erfolgt, dass die Konzentrationsveränderung des Gases über die Füllvorrichtung (12) größer oder kleiner als ein vorgegebener Grenzwert ist.
